# EUROPEAN PATENT APPLICATION

(11) **EP 2 233 496 A1**
(43) Date of publication of application: **29.09.2010**
(21) Application number: 09156324.7
(22) Date of filing: 26.03.2009
(51) Int. Cl.: C07K 14/005, C12N 9/88, C12N 15/34, C12N 15/60, G01N 33/58

(54) **Fluorescent proteins**

(71) Applicant: Ruhr-Universität Bochum, 44801 Bochum (DE)
(72) Inventor: Frankenberg-Dinkel, Nicole, 44789 Bochum (DE); Wiethaus, Jessica, 44263 Dortmund (DE); Scholte, Christian, 45239 Essen (DE)
(74) Representative: Viering, Jentschura & Partner

(57) **Abstract**

The present invention relates to fluorescent proteins composed of a viral phycobiliprotein lyase apoprotein and a tetrapyrrole chromophore and their use as fluorescence markers as well as nucleic acids encoding these proteins and methods for their production.

## Description

### Field of the Invention

The present invention relates to fluorescent proteins composed of a viral phycobiliprotein lyase apoprotein and a tetrapyrrole chromophore and their use as fluorescence markers as well as nucleic acids encoding these proteins and methods for their production.

### Background of the Invention

Phycobilisomes are the major light-harvesting complexes of cyanobacteria, rhodophytes and cryptophytes. These photosynthetic antenna proteins efficiently harvest light in the 'green gap' where chlorophylls absorb only poorly (Sidler, W.A., Phycobilisome and phycobiliprotein structures, in The Molecular Biology of Cyanobacteria. , B. D.A., Editor. 1994, Dordrecht: Kluwer. p. 139-216). This is achieved via open-chain (linear) tetrapyrrole chromophores, known as bilins that are covalently bound via thioether bonds to specific cysteine residues of the apoproteins.

Bilins are derived from heme, which is converted to the first open-chain product biliverdin IXα (BV IXα) by ferredoxin-dependent heme oxygenases (Frankenberg-Dinkel, N. and J.C. Lagarias, Biosynthesis and biological functions of bilins, in The porphyrin handbook, K.M. Kadish, K.M. Smith, and R. Guilard, Editors. 2003, Academic press: Amsderdam. p. 211-236; Dammeyer, T. and N. Frankenberg-Dinkel, Function and distribution of bilin biosynthesis enzymes in photosynthetic organisms. Photochem Photobiol Sci, 2008. 7(10): p. 1121-30). In cyanobacteria BV IXα is further metabolized by enzymes of the ferredoxin-dependent bilin reductase (FDBR) family. Two sequential two-electron reductions catalyzed by PebA and PebB yield phycoerythrobilin (PEB) (Dammeyer, T. and N. Frankenberg-Dinkel, Insights into phycoerythrobilin biosynthesis point toward metabolic channeling. J Biol Chem, 2006. 281(37): p. 27081-9). Alternatively, in a four-electron reduction BV IXα is reduced to phycocyanobilin (PCB) by the action of PcyA. In addition, cyanophages carry homologs of phycobilisome pigment biosynthesis genes (Dammeyer, T., et al., Efficient phage-mediated pigment biosynthesis in oceanic cyanobacteria. Curr Biol, 2008. 18(6): p. 442-8). Among these *pebS* is coding for a Phycoerythrobilin synthase. PebS is exclusive to phages and catalyzes a unique four-electron reduction of BV IXα to PEB (Dammeyer, T. and N. Frankenberg-Dinkel, Function and distribution of bilin biosynthesis enzymes in photosynthetic organisms. Photochem Photobiol Sci, 2008. 7(10): p. 1121-30).

Autocatalytic addition of PEB or PCB to phycobiliproteins is rare and in most cases regio- and stereoselective chromophore attachment is supported by phycobiliprotein lyases (Scheer, H. and K.H. Zhao, Biliprotein maturation: the chromophore attachment. Mol Microbiol, 2008. 68(2): p. 263-76). An increasing number of phycobiliprotein lyases has been identified in cyanobacteria but only few examples of the three known lyases types have been characterized in detail so far. E/F-type lyases catalyze chromophore addition to the cysteine α-84 of phycocyanins and phycoerythrocyanins and can have an additional isomerase activity. T-type lyases serve the cysteine β-155 binding site in phycocyanins and phycoerythrocyanins. The S/U-type in contrast acts as near-universal lyase for cysteine-84 binding sites in cyanobacterial phycobiliproteins.

After autocatalytic or lyase-mediated chromophore binding, all of the known bacterial phycobiliproteins show fluorescence. Several of these complexes are spectroscopically well characterized. However, the absorption or fluorescence characteristics of the lyasechromophore complexes have not been elucidated yet.

Fluorescent proteins are widely used for analysis of cells and molecules in numerous fluorescence-based assays and technologies and therefore fundamental tools in bioanalytic and clinical diagnostics (Shaner, N.C., P.A. Steinbach, and R.Y. Tsien, A guide to choosing fluorescent proteins. Nat Methods, 2005. 2(12): p. 905-9; Chudakov, D.M., S. Lukyanov, and K.A. Lukyanov, Fluorescent proteins as a toolkit for in vivo imaging. Trends Biotechnol, 2005. 23(12): p. 605-13; Shaner, N.C., G.H. Patterson, and M.W. Davidson, Advances in fluorescent protein technology. J Cell Sci, 2007. 120(Pt 24): p. 4247-60). The optical window for the visualization of proteins in living cells is limited by the autoabsorption and autofluorescence of the cell components. To minimize background signals, fluorescent protein markers with excitation and emission maxima in the red and near infrared region of the spectrum are highly desirable. Although some phycobiliproteins from rhodophytes and certain homologs of *Aequorea Victoria* green fluorescent protein (GFP) possess desirable excitation and emission maxima, their usefulness as fluorescent labels is limited due to their tendency to oligomerize, their high molecular weight and/or their weak fluorescence if recombinantly expressed. Additionally, it is difficult to express phycobiliproteins in heterologous systems, since they require a number of additional enzymes that facilitate chromophore synthesis and coupling.

Accordingly, there exists the need for stable, low molecular weight fluorescent proteins that exhibit strong fluorescence with excitation and emission maxima in the red and near infrared spectrum which are at the same time suitable for recombinant expression in heterologous expression systems. Although the number of available protein-based fluorescence markers has increased in the recent years, this need has not been met yet.

The fluorescent proteins of the present invention satisfy this long-felt need in the field.

### Summary of the Invention

The present invention is based on the inventors' finding that putative phycobiliprotein lyases from cyanophages, in particular the T-type phycobiliprotein lyase CpeT from the marine cyanophage Syn9, form stable, highly fluorescent complexes with tetrapyrrole chromophores, such as PEB.

Thus, in a first aspect, the present invention relates to an isolated polypeptide comprising, consisting essentially of or consisting of the amino acid sequence set forth in any one of SEQ ID Nos. 7-12 and mutants, variants and fragments thereof.

In another aspect, the present invention is directed to a fluorescent protein or fluorescent adduct comprising the isolated polypeptide of the invention and a chromophore moiety.

In a further aspect, the invention relates to a hybrid molecule comprising the polypeptide or the fluorescent protein of invention linked or coupled to a biomolecule.

In still another aspect, the invention relates to an isolated or synthesized nucleic acid molecule selected from the group consisting of:
(a) nucleic acid molecules comprising, consisting essentially of or consisting of the nucleotide sequence set forth in any one of SEQ ID Nos. 1-6 or complements thereof;
(b) nucleic acid molecules encoding a polypeptide sequence having the amino acid sequence set forth in any one of SEQ ID Nos. 7-12 or complements thereof;
(c) nucleic acid molecules encoding a polypeptide of the invention; and
(d) nucleic acid molecules comprising, consisting essentially of or consisting of a nucleotide sequence that has at least 50, 60, 70, 80, 90, 95, 98 or 99 % sequence identity to the nucleic acid molecules of (a) - (c).

In a still further aspect, the invention encompasses a vector comprising the nucleic acid molecule of the invention.

In another aspect, the invention also relates to a nucleic acid comprising:
(a) a first coding sequence selected from the group consisting of the nucleic acid molecules defined above under (a)-(d); and
(b) a second coding sequence that encodes a selected polypeptide,
   wherein the first coding sequence is fused to the second coding sequence such that expression of the fused sequence yields a hybrid protein in which the polypeptide encoded by the first coding sequence is fused to the polypeptide encoded by the second coding sequence.

In a further aspect, the invention is also directed to a host cell comprising the nucleic acid or the vector according to the invention.

In still another aspect, the invention relates to a method for preparing a recombinant polypeptide, comprising expressing the nucleic acid according to the invention in a host cell and obtaining therefrom the polypeptide.

The invention also features a method for the generation of a fluorescent protein, comprising contacting a polypeptide of the invention with a chromophore.

Also encompassed by the present invention is the use of the polypeptide or the fluorescent protein according to the invention as a detectable label or as a reporter for gene expression in cells. Such uses may include use of the polypeptide or fluorescent protein as a molecular switch or as a FRET reporter.

In a still further aspect, the invention is also directed to a method of detecting the presence of an analyte in a sample, comprising:
(a) contacting a sample with a detection reagent that specifically binds the analyte, wherein the detection reagent comprises a fluorescent protein according to the invention;
(b) exposing the sample to light that causes the fluorescent protein to emit light;
   and
(c) detecting the emitted light, thereby detecting the presence of the analyte.

In still another aspect, the present invention also relates to a detection reagent that comprises a polypeptide or fluorescent protein according to the invention.

In a further aspect, the invention also encompasses kits that comprise one or more of the group consisting of the polypeptides, fluorescent adducts, hybrid molecules, nucleic acids, vectors, host cells and detection reagents of the invention.

### Brief description of the drawings

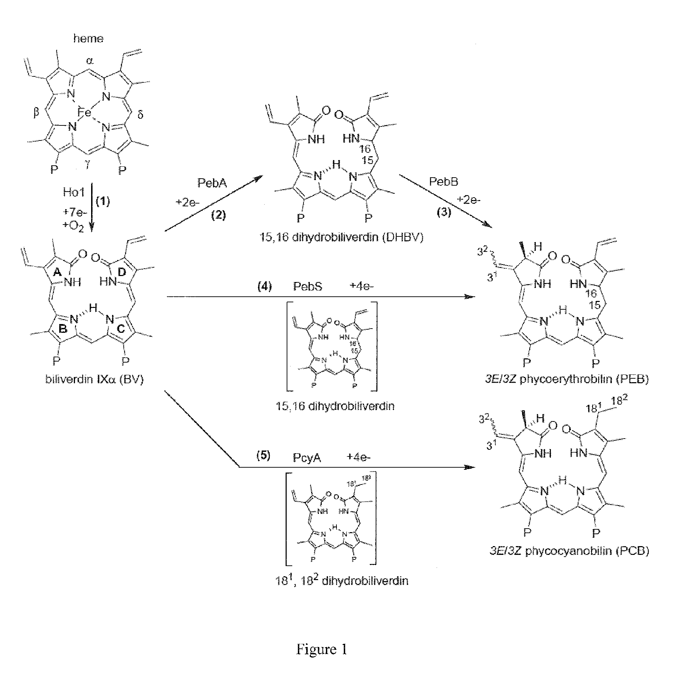

Figure 1 shows the biosynthesis pathway of open-chain tetrapyrroles in cyanobacteria and cyanophages. The first open-chain product biliverdin IXα (BV IXα) is derived from heme by a heme oxygenase-catalyzed reaction (1). BV IXα is the substrate for various enzymes of the FDBR family. Two sequential two-electron reductions catalyzed by PebA (2) and PebB (3) yield phycoerythrobilin (PEB). Phycoerythrobilin synthase (PebS) catalyzes a unique four-electron reduction of BV IXα to PEB (4). In a similar reaction, BV is reduced to phycocyanobilin (PCB) by the action of PcyA (5). All FDBRs obtain the required number of electrons from the redox cofactor ferredoxin. Enzymes catalyzing reactions 2 and 3 are found in cyanobacterial host cells. Enzymes catalyzing reactions 1 and 5 are found in both cyanobacterial host cells and cyanophages. The PebS enzyme (4) is found solely in cyanophage populations. The following abbreviations are used: propionate side chain (P), heme oxygenase (Ho1), PCB:ferredoxin oxidoreductase (PcyA), 15,16-DHBV:ferredoxin oxidoreductase (PebA), PEB:ferredoxin oxidoreductase (PebB), and PEB synthase (PebS).

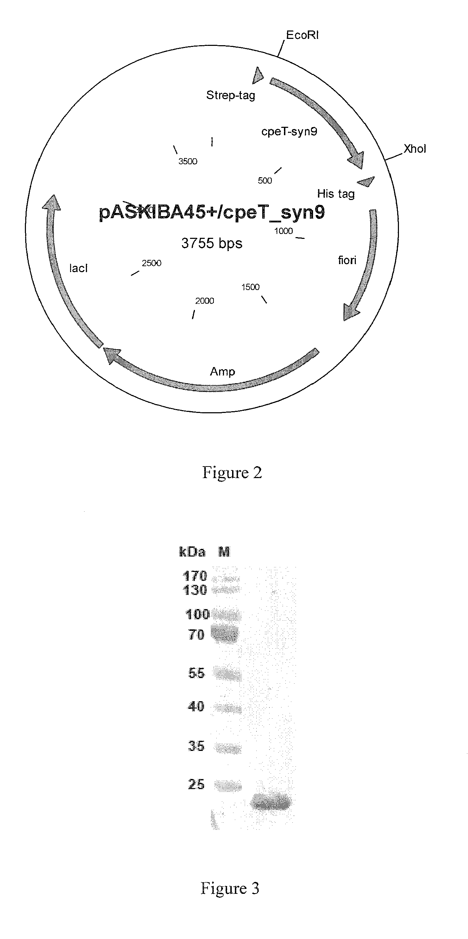

Figure 2 shows a map of the expression vector pASKIBA45+ in which the *cpeT* gene from cyanophage Syn9 (SEQ ID NO:1) was cloned as an EcoRI/XhoI fragment, resulting in expression plasmid pASKIBA45+/cpeT_syn9.
Figure 3 shows the affinity purification of the lyase CpeT from cyanophage Syn9. The lyase was recombinantly produced in *Escherichia coli* with an N-terminal *Strep*-tag® and purified via affinity chromatography. Purified protein was analyzed by SDS-PAGE. The PageRuler prestained protein ladder (Fermentas, St. Leon-Rot, Germany) was used as a molecular weight standard (M).

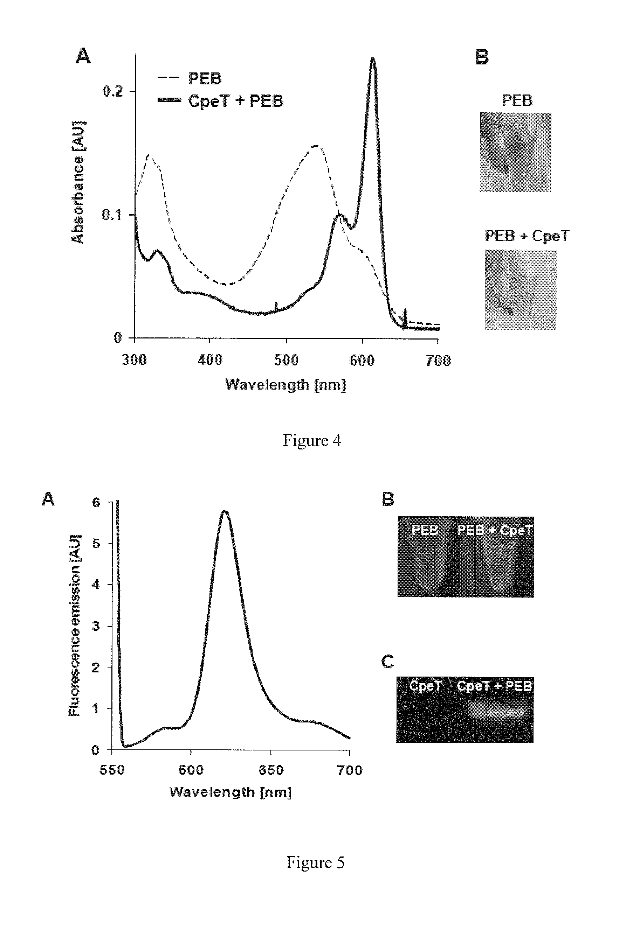

Figure 4 shows the absorption properties of CpeT:PEB. Incubation of CpeT with PEB (1:1 ratio) resulted nearly immediately in a complex with an absorption maximum at 612 nm (A) and a blue color (B).
Figure 5 shows the fluorescence properties of CpeT:PEB. PEB-chromophorylated CpeT exhibited a fluorescence emission maximum at 621 nm after excitation at 550 nm (A). Under U/V light and on zinc-induced red fluorescence blots neither free bilin (B, left) nor free lyase (C, left) exhibited fluorescence compared to CpeT:PEB (B and C, right).

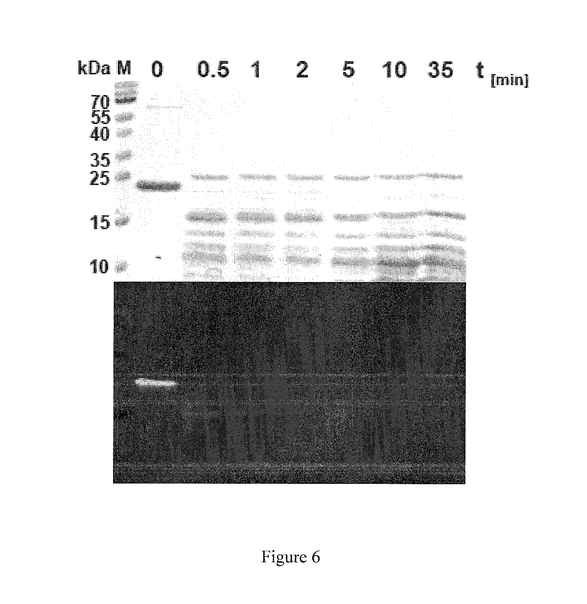

Figure 6 shows the analysis of CpeT:PEB digested with trypsin. During incubation of the CpeT:PEB complex with trypsin, samples were taken at the indicated time points and analyzed in a Coomassie stained SDS-Gel (top) and in a zinc-induced red fluorescence blot (bottom). The PageRuler prestained protein ladder (Fermentas, St. Leon-Rot, Germany) was used as a molecular weight standard (M).

### Detailed Description

### Definitions

Unless otherwise stated the following terms used in the specification and claims have the meanings discussed below:
The terms "polynucleotide" and "nucleic acid (molecule)" are used interchangeably to refer to polymeric forms of nucleotides of any length, including naturally occurring and non-naturally occurring nucleic acids. The polynucleotides may contain deoxyribonucleotides, ribonucleotides and/or their analogs. The term "nucleic acid molecule" includes single-, double-stranded and triple helical molecules. The following are non-limiting embodiments of nucleic acids: a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes and primers. A nucleic acid molecule may also comprise modified nucleic acid molecules, such as methylated nucleic acid molecules and nucleic acid molecule analogs. Analogs of purines and pyrimidines are known in the art, and include, but are not limited to, aziridinylcytosine, 4-acetylcytosine, 5-fluorouracil, 5-bromouracil, 5-carboxymethylaminomethyl-2-thiouracil, 5-carboxymethyl-aminomethyluracil, inosine, N6-isopentenyladenine, 1-methyladenine, 1- methylpseudouracil, 1-methylguanine, 1-methylinosine, 2,2-dimethylguanine, 2-methyladenine, 2-methylguanine,3-methylcytosine,5-methylcytosine, pseudouracil, 5-pentylnyluracil and 2,6-diaminopurine.
The use of uracil as a substitute for thymine in a deoxyribonucleic acid is also considered an analogous form of pyrimidine.

"Oligonucleotide" refers to polynucleotides of between 5 and about 100 nucleotides of single- or double-stranded nucleic acid, typically DNA. Oligonucleotides are also known as oligomers or oligos and may be isolated from genes, or chemically synthesized by methods known in the art.

A "primer" refers to an oligonucleotide, usually single-stranded, that provides a 3'-hydroxyl end for the initiation of enzyme-mediated nucleic acid synthesis.

By "isolated" in reference to nucleic acid is meant a polymer of nucleotides conjugated to each other, including DNA and RNA, that is isolated from a natural source or that is synthesized. The isolated nucleic acids of the present invention are not found in a pure or separated state in nature. Use of the term "isolated" indicates that a naturally occurring sequence has been removed from its normal cellular (*i*.*e*., chromosomal) environment. Thus, the sequence may be in a cell-free solution or placed in a different cellular environment. The term does not imply that the sequence is the only nucleotide chain present, but that it is essentially free (about 90 - 95% pure at least) of non-nucleotide material naturally associated with it, and thus is distinguished from isolated chromosomes.

It is also advantageous for some purposes that a nucleotide sequence be present in purified form. The term "purified" in reference to nucleic acid does not require absolute purity (such as a homogeneous preparation). Instead, it represents an indication that the sequence is relatively more pure than in the natural environment (compared to the natural level this level should be at least 2-5 fold greater, *e.g*., in terms of mg/ml). Individual clones isolated from a cDNA library may be purified to electrophoretic homogeneity. The claimed DNA molecules obtained from these clones could be obtained directly from total DNA or from total RNA. The cDNA clones are not naturally occurring, but rather are preferably obtained via manipulation of a partially purified naturally occurring substance (messenger RNA). The construction of a cDNA library from mRNA involves the creation of a synthetic substance (cDNA) and pure individual cDNA clones can be isolated from the synthetic library by clonal selection of the cells carrying the cDNA library. Thus, the process which includes the construction of a cDNA library from mRNA and isolation of distinct cDNA clones yields an approximately 10⁶-fold purification of the native message. Thus, purification of at least one order of magnitude, preferably two or three orders, and more preferably four or five orders of magnitude is expressly contemplated.

The term "complementary" or "complement" refers to two nucleotides that can form multiple favourable interactions with one another. Such favourable interactions include Watson-Crick base pairing. A nucleotide sequence is the complement of another nucleotide sequence if all of the nucleotides of the first sequence are complementary to all of the nucleotides of the second sequence. "Substantially complementary" as used herein refers to the fact that a given nucleic acid molecule is at least 90, at least 95, or 99 or 100% complementary to another nucleic acid.

The term "vector" relates to a single or double-stranded circular nucleic acid molecule that can be transfected into cells and replicated within or independently of a cell genome. A circular double-stranded nucleic acid molecule can be cut and thereby linearized upon treatment with restriction enzymes. An assortment of nucleic acid vectors, restriction enzymes, and the knowledge of the nucleotide sequences cut by restriction enzymes are readily available to those skilled in the art. A nucleic acid molecule encoding a polypeptide of the invention can be inserted into a vector by cutting the vector with restriction enzymes and ligating the two pieces together.

The term "promoter" as used herein, refers to nucleic acid sequence needed for gene sequence expression. Promoter regions vary from organism to organism, but are well known to persons skilled in the art for different organisms. For example, in prokaryotes, the promoter region contains both the promoter (which directs the initiation of RNA transcription) as well as the DNA sequences which, when transcribed into RNA, will signal synthesis initiation. Such regions will normally include those 5'-non-coding sequences involved with initiation of transcription and translation, such as the TATA box, capping sequence, CAAT sequence, and the like.

By "exogenous" in reference to a promoter, it is meant that the respective promoter is not normally coupled *in vivo* transcriptionally to the coding sequence for the polypeptide of interest.

A nucleic acid molecule, such as DNA, is said to be "capable of expressing" a polypeptide if it contains nucleotide sequences which contain transcriptional and translational regulatory information and such sequences are "operably linked" to nucleotide sequences which encode the polypeptide. An operable linkage is a linkage in which the regulatory DNA sequences and the DNA sequence sought to be expressed are connected in such a way as to permit gene sequence expression. The precise nature of the regulatory regions needed for gene sequence expression may vary from organism to organism, but shall in general include a promoter region which, in prokaryotes, contains both the promoter (which directs the initiation of RNA transcription) as well as the DNA sequences which, when transcribed into RNA, will signal synthesis initiation. Such regions will normally include those 5'-non-coding sequences involved with initiation of transcription and translation, such as the TATA box, capping sequence, CAAT sequence, and the like.

Two DNA sequences (such as a promoter region sequence and a sequence encoding a polypeptide) are said to be operably linked if the nature of the linkage between the two DNA sequences does not (1) result in the introduction of a frame-shift mutation, (2) interfere with the ability of the promoter region sequence to direct the transcription of a gene sequence encoding the polypeptide, or (3) interfere with the ability of the gene sequence of a polypeptide to be transcribed by the promoter region sequence.

"Peptide" generally refers to a short chain of amino acids linked by peptide bonds. Typically peptides comprise amino acid chains of about 2-20 amino acids. "Polypeptide" generally refers to individual straight or branched chain sequences of amino acids that are typically longer than peptides. "Polypeptides" usually comprise at least about 30 to 1000 amino acids in length, more typically at least about 50 to 600 amino acids, and frequently at least about 100 to about 500 amino acids. "Proteins" include single polypeptides as well as complexes of multiple polypeptide chains, which may be the same or different.

Multiple chains in a protein may be characterized by secondary, tertiary and quaternary structure as well as the primary amino acid sequence structure, may be held together, for example, by disulfide bonds, and may include post-synthetic modifications such as, without limitation, glycosylation, phosphorylation, truncations or other processing.

By "isolated" in reference to a polypeptide is meant a polymer of amino acids (2 or more amino acids) conjugated to each other, including polypeptides that are isolated from a natural source or that are synthesized. The isolated polypeptides of the present invention are unique in the sense that they are not found in a pure or separated state in nature. Use of the term "isolated" indicates that a naturally occurring sequence has been removed from its normal cellular environment. Thus, the sequence may be in a cell-free solution or placed in a different cellular environment. The term does not imply that the sequence is the only amino acid chain present, but that it is essentially free (about 90 - 95% pure at least) of non-amino acid material naturally associated with it.

By the use of the term "enriched" in reference to a polypeptide is meant that the specific amino acid sequence constitutes a significantly higher fraction (2 - 5 fold) of the total amino acid sequences present in the cells or solution of interest than in normal or diseased cells or in the cells from which the sequence was taken. This could be caused by preferential reduction in the amount of other amino acid sequences present, or by a preferential increase in the amount of the specific amino acid sequence of interest, or by a combination of the two. However, it should be noted that enriched does not imply that there are no other amino acid sequences present, just that the relative amount of the sequence of interest has been significantly increased. The term significant here is used to indicate that the level of increase is useful to the person making such an increase, and generally means an increase relative to other amino acid sequences of about at least 2-fold, more preferably at least 5- to 10-fold or even more. The term also does not imply that there is no amino acid sequence from other sources. The other source of amino acid sequences may, for example, comprise amino acid sequence encoded by a yeast or bacterial genome, or a cloning vector. The term is meant to cover only those situations in which man has intervened to increase the proportion of the desired amino acid sequence.

It is also advantageous for some purposes that an amino acid sequence be in purified form. The term "purified" in reference to a polypeptide does not require absolute purity (such as a homogeneous preparation); instead, it represents an indication that the sequence is relatively purer than in the natural environment. Compared to the natural level this level should be at least 2-5 fold greater (*e.g*., in terms of mg/ml). Purification of at least one order of magnitude, preferably two or three orders, and more preferably four or five orders of magnitude is expressly contemplated. The substance is preferably free of contamination at a functionally significant level, for example 90%, 95%, or 99% pure.

By "fragment" in reference to a polypeptide is meant any amino acid sequence present in a polypeptide, as long as it is shorter than the full length sequence.

By "recombinant polypeptide" is meant a polypeptide produced by recombinant DNA techniques such that it is distinct from a naturally occurring polypeptide either in its location (*e.g*., present in a different cell or tissue than found in nature), purity or structure. Generally, such a recombinant polypeptide will be present in a cell in an amount different from that normally observed in nature.

The term "mutated" or "mutant" in reference to a nucleic acid or a polypeptide refers to the exchange, deletion, or insertion of one or more nucleotides or amino acids, respectively, compared to the naturally occurring nucleic acid or polypeptide.

The term "altered" or "variant" in reference to a nucleic acid or polypeptide refers to polymorphisms, i.e. the exchange, deletion, or insertion of one or more nucleotides or amino acids, respectively, compared to the predominant form of the respective nucleic acid or polypeptide. Such an alteration may include single nucleotide polymorphisms (SNPs).

The term "adduct" as used herein relates to a product of a direct addition of two or more distinct molecules, resulting in a single reaction product containing all atoms of all components. In the adduct of the invention, the polypeptide of the invention is bound to or complexed with a chromophore. This binding can be covalent or non-covalent.

"Fluorescent protein" or "fluorescent adduct" as used interchangeably herein relates to the polypeptide of the invention complexed with or bound to a chromophore moiety. In certain embodiments of the invention, this chromophore is a bilin, for example a phycobilin, such as PEB, and the binding may be covalent or non-covalent.

By "identity" is meant a property of sequences that measures their similarity or relationship. Identity is measured by dividing the number of identical residues by the total number of residues and gaps and multiplying the product by 100.

"Gaps" are spaces in an alignment that are the result of additions or deletions of amino acids or nucleotides. Thus, two copies of exactly the same sequence have 100% identity, but sequences that are less highly conserved, and have deletions, additions, or replacements, may have a lower degree of identity. Those skilled in the art will recognize that several computer programs are available for determining sequence identity using standard parameters, for example Blast (Altschul, et al. (1997) Nucleic Acids Res. 25:3389-3402), Blast2 (Altschul, et al. (1990) J. mol. biol. 215:403-410), and Smith-Waterman (Smith, et al. (1981) J. Mol. Biol. 147:195-197).

The term "mammalian" refers to any mammal, for example species such as mice, rats, rabbits, guinea pigs, sheep, and goats, cats, dogs, monkeys, apes, and humans.

The term "complex" refers to an assembly of at least two molecules bound to one another.

The term "expressing" as used herein refers to the production of a polypeptide from a nucleic acid vector containing a nucleic acid sequence encoding the polypeptide within a cell. The nucleic acid vector is transfected into cells using well-known techniques in the art as described herein.

The term "transfecting" defines a number of methods to insert a nucleic acid vector or other nucleic acid molecules into a cellular organism. These methods involve a variety of techniques, such as treating the cells with high concentrations of salt, elevated temperature, an electric field, detergent, or DMSO to render the outer membrane or wall of the cells permeable to nucleic acid molecules of interest or use of various viral transduction strategies.

The terms "contacting" or "incubating" as used interchangeably herein refer generally to providing access of one component, reagent, analyte or sample to another.

The term "detecting" as used herein refers to any method of verifying the presence of a given molecule.

The term "analyte" as used herein, refers to any substance that can be detected in an assay by binding to a detection reagent, and which may be present in a sample. Therefore, the analyte can be, without limitation, any substance for which there exists a naturally occurring antibody or for which an antibody can be prepared. The analyte may, for example, be a protein, a polypeptide, a hapten, a carbohydrate, a nucleic acid, a lipid, a cell or any other of a wide variety of biological or non-biological molecules, complexes or combinations thereof. Generally, the analyte will be a protein, peptide, carbohydrate, nucleic acid or lipid derived from a biological source such as bacterial, fungal, viral, plant or animal samples. Additionally, however, the target may also be a smaller organic compound such as a drug, drug-metabolite, dye or other small molecule present in the sample.

The term "sample", as used herein, refers to an aliquot of material, frequently biological matrices, an aqueous solution or an aqueous suspension derived from biological material. Samples to be assayed for the presence of an analyte by the methods of the present invention include, for example, cells, tissues, homogenates, lysates, extracts, and purified or partially purified proteins and other biological molecules and mixtures thereof. Further non-limiting examples of samples typically used include human and animal body fluids such as whole blood, serum, plasma, cerebrospinal fluid, sputum, bronchial washing, bronchial aspirates, urine, semen, lymph fluids and various external secretions of the respiratory, intestinal and genitourinary tracts, tears, saliva, milk, white blood cells, myelomas and the like; biological fluids such as cell culture supernatants; tissue specimens which may or may not be fixed; and cell specimens which may or may not be fixed. The samples used in the methods of the present invention will vary based on the assay format and the nature of the tissues, cells, extracts or other materials, especially biological materials, to be assayed. Methods for preparing protein extracts from cells or samples are well known in the art and can be readily adapted in order to obtain a sample that is compatible with the methods of the invention

"Specifically binding" and "specific binding" as used herein mean that the binding molecule binds to the analyte based on specific recognition of a binding region or epitope on the analyte. The binding molecule preferably recognizes and binds to the target molecule with a higher binding affinity than it binds to other components present in the sample. In various embodiments of the invention, "specifically binding" may mean that an antibody or other biological molecule, binds to a target molecule with at least about a 10⁶-fold greater affinity, preferably at least about a 10⁷-fold greater affinity, more preferably at least about a 10⁸-fold greater affinity, and most preferably at least about a 10⁹-fold greater affinity than it binds molecules unrelated to the target molecule. Typically, specific binding refers to affinities in the range of about 10⁶-fold to about 10⁹-fold greater than non-specific binding. In some embodiments, specific binding may be characterized by affinities greater than 10⁹-fold over non-specific binding. In a specific embodiment, the binding molecule uniquely recognizes and binds to the target molecule.

The term "antibody" is used herein in the broadest sense and specifically covers monoclonal and polyclonal antibodies as well as antibody fragments (e.g., Fab, F(ab')₂, scFv, Fv diabodies and linear antibodies), so long as they exhibit the desired binding activity. For a review of scFv see Pluckthun (1994) The Pharmacology of Monoclonal Antibodies, Vol. 113. Rosenburg and Moore eds. Springer-Verlag, New York, pp. 269-315. Diabodies are described more fully in, for example, European patent 404097, international patent publication WO 93/11161 and Hollinger et al. (1993) Proc. Natl. Acad. Sci. USA 90: 6444-6448. Linear antibodies are described in Zapata et al. (1995) Protein Eng. 8(10): 1057-1062.

The term "polyclonal" refers to antibodies that are heterogenous populations of antibody molecules derived from the sera of animals immunized with an antigen or an antigenic functional derivative thereof. For the production of polyclonal antibodies, various host animals may be immunized by injection with the antigen. Various adjuvants may be used to increase the immunological response, depending on the host species.

"Monoclonal antibodies" are substantially homogenous populations of antibodies to a particular antigen. They may be obtained by any technique which provides for the production of antibody molecules by continuous cell lines in culture. Monoclonal antibodies may be obtained by methods well known to those skilled in the art (see for example, Köhler et al., Nature 256:495-497, 1975, and U.S. Patent No. 4,376,110).

"Chromophore" as used herein relates to a moiety or molecule that absorbs certain wavelengths of light, usually visible light, and transmits or reflects others, so that the molecule has a color. These characteristics may also be imparted or influenced by the binding of the chromophore to another molecule. The chromophore may be fluorescent in that it absorbs a certain wavelength of light and emits another wavelength of light. Non-limiting examples for chromophores are bilirubin, heme, chlorophyll, phycoerythrobilin, phycocyanobilin, phytochromobilin, phycourobilin, phycoviolobilin, dihydrobiliverdin, biliverdin.

"Derivative" in reference to a chromophore relates to a compound that is formed from a similar known chromophore by replacing one atom or a group of atoms of the original structure with another atom or group of atoms. Such a modification can, for example, be obtained by means of organic chemistry known to the person skilled in the art.

"Biomolecule", as used herein, relates to molecules that belong to a class of naturally occurring molecules, such as proteins, polypeptides, peptides, carbohydrates, lipids or nucleic acids.

### Embodiments of the invention

### Polypeptides

The present invention is directed to isolated polypeptides comprising, consisting essentially of or consisting of the amino acid sequence set forth in any one of SEQ ID Nos. 7-12.

Explicitly falling within the scope of the present invention are fragments of the polypeptides having any one of the amino acid sequences set forth in SEQ ID Nos. 7-12. The fragments may, for example, be N-terminally and/or C-terminally truncated polypeptides of SEQ ID Nos. 7-12. In one embodiment, the fragments comprise, consist essentially of or consist of at least 30, 35, 40, 45, 50, 60, 70, 80, 90, 100, 120, or 150 contiguous amino acids of SEQ ID Nos. 7-12. In one preferred embodiment of the invention, the above fragments retain their ability to bind a chromophore.

In one embodiment, the present invention is thus directed to polypeptides comprising, consisting essentially of or consisting of about 15-150, 20-120, 30-100 or 40-80 amino acids of the amino acid sequence set forth in any one of SEQ ID Nos. 7-12 and mutants, variants and fragments thereof.

Also encompassed by the present invention are variants of any one of the amino acid sequences set forth in SEQ ID Nos. 7-12 and fragments thereof. Such fragments of variant polypeptides may have a length of at least 30, 35, 40, 45, 50, 60, 70, 80, 90, 100, 120, or 150 amino acids. In preferred embodiments of the invention, the variants retain their ability to bind a chromophore.

Also intended to fall within the scope of the present invention, are splice variants of the above polypeptides. Said splice variants may significantly differ from the above amino acid sequences, however, the functional domain architecture is retained in such variants. Such splice variants may lead to isoforms of the polypeptides of the invention and may differ from the known form, for example, by an extended or shortened C- or N-terminus or the insertion or deletion of an amino acid sequence stretch. However, the sequence homology and sequence identity between the splice variants/isoforms is sufficiently high so that the skilled person is readily aware that the polypeptide in question is a mere isoform and not another protein of the same family.

The present invention also includes mutants of the above polypeptides. The mutation may be a deletion, insertion or substitution of one or more, for example 1, 2, 3, 4 or 5 amino acids. In preferred embodiments of the invention, the mutants also retain their ability to bind a chromophore.

The present invention thus also relates to polypeptides comprising an amino acid sequence that is about 60, about 70, about 80, about 90, about 95, about 97, about 98, or about 99 % identical to the amino acid sequences set forth in any one of SEQ ID Nos. 7-12.

The polypeptides according to the invention may be derived from a cyanophage, i.e. a virus infecting cyanobacteria, preferably marine cyanobacteria. In one embodiment, the phage is selected from the group consisting of cyanophage Syn9, cyanophage Syn1, cyanophage Syn19, cyanophage S-PM2, cyanophage S-SSM5 and cyanophage P-RSM4.

In one embodiment of the invention, the polypeptides have sequence homology to phycobiliprotein lyases, are putative phycobiliprotein lyases or actually have phycobiliprotein lyase activity. In one embodiment of the invention, the polypeptides are (putative) T-type phycobiliprotein lyases, for example CpeT.

The polypeptides of the inventions may be apoproteins, i.e. the protein component of an enzyme that includes a cofactor, i.e. a non-proteinaceous chemical compound. The cofactor may be a chromophore.

Hence, the present invention also includes adducts that comprise the polypeptides of the invention and a chromophore moiety. In one embodiment of the invention the chromophore moiety is a tetrapyrrole, for example an open chain tetrapyrrole. In a specific embodiment, the chromophore is a bilin, for example a phycobilin. The chromophore may be selected from the group consisting of phycoerythrobilin, phycocyanobilin, phytochromobilin, phycourobilin, phycoviolobilin, dihydrobiliverdin, biliverdin, bilirubin and derivatives thereof.

The chromophore may be covalently or non-covalently bound to the polypeptide. If non-covalently bound, the binding may be facilitated by hydrogen bonding, ion pairing and/or van der Waals forces.

In specific embodiments of the invention the fluorescent proteins may absorb light with a wavelength in the visible spectrum, for example between about 350 and about 700 nm. In another embodiment, the fluorescent adducts of the invention emit light of a wavelength between about 350 and about 700 nm. In preferred embodiments of the invention, the fluorescent adducts have excitation and/or emission spectrum maxima in the red or near infrared region of the spectrum, i.e. between about 600 and about 700 nm. In one particularly preferred embodiment, the fluorescent adduct has an excitation spectrum maximum at about 610 - 615 nm and/or an emission spectrum maximum at about 620 - 625 nm.

The polypeptides and fluorescent adducts of the invention may be linked to another molecule, for example a biomolecule, such as a protein, polypeptide, peptide, carbohydrate, lipid or nucleic acid. In one embodiment, the biomolecule is a polypeptide or protein selected from the group consisting of a signalling protein, a cell-surface receptor, an enzyme, an antibody, an antibody fragment or an antibody-like molecule. In another embodiment, the biomolecule may be a nucleic acid, such as an oligonucleotide (probe).

Accordingly, the present invention also encompasses hybrid molecules comprising a polypeptide or fluorescent adduct of the invention linked or fused to a biomolecule, as defined above. The linkage may be a covalent bond, for example a peptide bond. The hybrid molecule may be a hybrid protein or a fusion protein. If the hybrid molecule is a hybrid protein, the coupling may be achieved by recombinantly expressing a nucleic acid construct encoding the complete hybrid protein/fusion protein. Alternatively, the coupling of the polypeptides/fluorescent adducts of the invention with any of the above mentioned molecules may be achieved by organic chemistry.

If the hybrid molecule is a fusion protein, the polypeptides or fluorescent adducts of the invention may be fused N- or C-terminally to the protein of interest. Such recombinant fusion proteins may be generated by expressing a specifically designed nucleic acid construct that encodes the fusion protein in a host cell and isolating the thus generated recombinant protein.

### Nucleic acids

The present invention is also directed to an isolated or synthesized nucleic acid molecule selected from the group consisting of:
(a) nucleic acid molecules comprising, consisting essentially of or consisting of the nucleotide sequence set forth in any one of SEQ ID Nos. 1-6 or complements thereof;
(b) nucleic acid molecules encoding a polypeptide sequence having the amino acid sequence set forth in any one of SEQ ID Nos. 7-12 or complements thereof;
(c) nucleic acid molecules encoding a polypeptide of the invention; and
(d) nucleic acid molecules comprising, consisting essentially of or consisting of a nucleotide sequence that has at least 50, 60, 70, 80, 90, 95, 98 or 99 % sequence identity to the nucleic acid molecules of (a) - (c).

Also encompassed by the present invention are nucleic acids that comprise:
(a) a first coding sequence selected from the group consisting of the nucleic acid molecules according to the invention; and
(b) a second coding sequence that encodes a selected polypeptide,
   wherein the first coding sequence is fused to the second coding sequence such that expression of the fused sequence yields a hybrid protein in which the polypeptide encoded by the first coding sequence is fused to the polypeptide encoded by the second coding sequence.

In these nucleic acid molecules, the first coding sequence preferentially encodes a polypeptide according to the invention, whereas the second coding sequence encodes a polypeptide different from those of the present invention. The second polypeptide may be any polypeptide or protein, for example an antibody or cell signalling molecule, a cell surface receptor, or a peptide or protein tag that facilitates purification of the polypeptide of the invention, for example streptavidin or a streptavidin fragment, glutathione-S-transferase, a 6x His tag etc.

Methods for selection and preparation of nucleic acids are diverse and well described in standard biomolecular protocols. A typical way would be preparative PCR and chromatographic purification starting from existing template DNAs or stepwise synthesis of artificial nucleic acids.

Also encompassed by the present invention are nucleic acid molecules substantially complementary to the above nucleic acid molecules in that they are at least 90, at least 95, or 99 or 100% complementary to any one of the above nucleic acid.

The nucleic acids according to the invention may be isolated from a natural source by cDNA cloning or subtractive hybridization or other routine techniques known to a person skilled in the art. The natural source may be any organism, but is preferably of viral or bacterial origin.

The nucleic acids of the invention may also be synthetic, meaning being synthesized by the triester method or by using an automated DNA synthesizer.

Included within the scope of this invention are also the functional equivalents of the herein-described isolated nucleic acid molecules. The degeneracy of the genetic code permits substitution of certain codons by other codons that specify the same amino acid and hence would give rise to the same protein. The nucleic acid sequence can vary substantially since, with the exception of methionine and tryptophane, the known amino acids can be coded for by more than one codon. Thus, portions or all of the polypeptide genes of the invention could be synthesized to give a nucleic acid sequence significantly different from that shown in SEQ ID Nos. 1-6. The encoded amino acid sequence thereof would, however, be preserved.

In addition, the nucleic acid sequence may comprise a nucleotide sequence which results from the addition, deletion or substitution of at least one nucleotide to the 5'-end and/or the 3'-end of the nucleic acid sequence set forth in any one of SEQ ID Nos. 1-6 or a derivative thereof. Any nucleotide or polynucleotide may be used in this regard, provided that its addition, deletion or substitution does not alter the amino acid sequence encoded by the nucleotide sequence. For example, the present invention is intended to include any nucleic acid sequence resulting from the addition of ATG as an initiation codon at the 5'-end of the inventive nucleic acid sequence or its derivative, or from the addition of TTA, TAG or TGA as a termination codon at the 3'-end of the inventive nucleotide sequence or its derivative. Moreover, the nucleic acid molecule of the present invention may, as necessary, have restriction endonuclease recognition sites added to its 5'-end and/or 3'-end.

Such functional alterations of a given nucleic acid sequence afford an opportunity to promote secretion and/or processing of heterologous proteins encoded by foreign nucleic acid sequences fused thereto. All variations of the nucleotide sequence of the polypeptide genes of the invention and fragments thereof permitted by the genetic code are, therefore, included in this invention.

Further, it is possible to delete codons or to substitute one or more codons with codons other than degenerate codons to produce a structurally modified polypeptide, but one which has substantially the same utility or activity as the polypeptide produced by the unmodified nucleic acid molecule. As recognized in the art, the two polypeptides are functionally equivalent, as are the two nucleic acid molecules that give rise to their production, even though the differences between the nucleic acid molecules are not related to the degeneracy of the genetic code.

The instant invention is intended to also encompass nucleic acids of recombinant origin, preferably in a cell or an organism.

Therefore, the present invention also relates to a recombinant DNA molecule comprising, 5' to 3', a promoter effective to initiate transcription in a host cell and the above-described nucleic acid molecules. In addition, the present invention relates to a recombinant DNA molecule comprising a vector and an above-described nucleic acid molecule. The recombinant nucleic acid can contain a transcriptional initiation region functional in a cell, a sequence complementary to an RNA sequence encoding a polypeptide of the invention and a transcriptional termination region functional in a cell. The above-described molecules may be isolated and/or purified DNA molecules.

The above nucleic acid molecules of the invention may be comprised within a vector. Such a vector may comprise a promoter. The promoter is preferably operably linked to the nucleic acid molecule and/or effective to initiate transcription in a host cell.

A promoter region is operably linked to a DNA sequence if the promoter is capable of effecting transcription of that DNA sequence. Thus, to express a gene encoding a polypeptide of the invention, transcriptional and translational signals recognized by an appropriate host are necessary.

Specific vectors and host cell combinations are discussed below.

### Host cells

The invention further describes a recombinant cell or tissue comprising a nucleic acid molecule according to the invention, as detailed above.

In such cells, the nucleic acid may be under the control of the genomic regulatory elements, or may be under the control of exogenous regulatory elements including an exogenous promoter.

The present invention further relates to a host cell or organism that contains an above-described nucleic acid molecule or vector and thereby is capable of expressing a polypeptide.

The polypeptide may be purified from cells which have been altered to express the polypeptide. A cell is said to be "altered to express a desired polypeptide" when the cell, through genetic manipulation, is made to produce a protein which it normally does not produce or which the cell normally produces at lower levels. One skilled in the art can readily adapt procedures for introducing and expressing either genomic, cDNA, or synthetic sequences into either eukaryotic or prokaryotic cells.

The present invention encompasses the expression of a gene encoding polypeptide of the invention (or a functional derivative thereof) in either prokaryotic or eukaryotic cells. Prokaryotic hosts are, generally, very efficient and convenient for the production of recombinant proteins and are, therefore, one type of preferred expression system for polypeptides of the invention. Prokaryotes most frequently are represented by various strains of *E. coli.* Other recognized prokaryotic hosts include bacteria such as *Bacillus, Streptomyces, Pseudomonas, Salmonella, Serratia*, and the like. The prokaryotic host must be compatible with the replicon and control sequences in the expression plasmid.

In one embodiment of the invention, the host cell is *Escherichia coli* or *Bacillus subtilis.*

In prokaryotic systems, plasmid vectors that contain replication sites and control sequences derived from a species compatible with the host may be used. Exemplary prokaryotic vectors include plasmids such as those capable of replication in *E. coli* (such as, for example, pBR322, ColEl, pSC101, pACYC 184, πVX; "Molecular Cloning: A Laboratory Manual", 1989, supra). Bacillus plasmids include pC194, pC221, pT127, and the like (Gryczan, In: The Molecular Biology of the Bacilli, Academic Press, NY, pp. 307-329, 1982). Suitable *Streptomyces* plasmids include p1J101 (Kendall et al., J. Bacteriol. 169:4177-4183, 1987), and streptomyces bacteriophages such as φC31 (Chater et al., In: Sixth International Symposium on Actinomycetales Biology, Akademiai Kaido, Budapest, Hungary, pp. 45-54, 1986). *Pseudomonas* plasmids are reviewed by John et al. (Rev. Infect. Dis. 8:693-704, 1986), and Izaki (Jpn. J. Bacteriol. 33:729-742, 1978). Suitable phage or bacteriophage vectors may include γgt10, γgt11 and the like; and suitable virus vectors may include pMAM-neo, pKRC and the like. Preferably, the selected vector of the present invention has the capacity to replicate in the selected host cell. In one embodiment, the vector used is pASKIBA45+ (IBA GmbH, Goettingen, Germany).

To express a polypeptide of the invention (or a functional derivative thereof) in a prokaryotic cell, it is necessary to operably link the sequence encoding the polypeptide of the invention to a functional prokaryotic promoter. Such promoters may be either constitutive or, more preferably, regulatable (*i*.*e*., inducible or derepressible). Examples of constitutive promoters include the *int* promoter of bacteriophage λ, the *bla* promoter of the β-lactamase gene sequence of pBR322, and the *cat* promoter of the chloramphenicol acetyl transferase gene sequence of pPR325, and the like. Examples of inducible prokaryotic promoters include the major right and left promoters of bacteriophage λ (P_{L} and P_{R}), the *trp*, *recA, lacZ, lacI*, and *gal* promoters of *E. coli*, the α-amylase (Ulmanen et al., J. Bacteriol. 162:176-182, 1985) and the sigma-28-specific promoters of *B. subtilis* (Gilman et al., Gene Sequence 32:11-20, 1984), the promoters of the bacteriophages of *Bacillus* (Gryczan, In: The Molecular Biology of the Bacilli, Academic Press, Inc., NY, 1982), and *Streptomyces* promoters (Ward et al., Mol. Gen. Genet. 203:468-478, 1986). Prokaryotic promoters are reviewed by Glick (Ind. Microbiol. 1:277-282, 1987), Cenatiempo (Biochimie 68:505-516, 1986), and Gottesman (Ann. Rev. Genet. 18:415-442, 1984).

Proper expression in a prokaryotic cell also requires the presence of a ribosome-binding site upstream of the gene sequence-encoding sequence. Such ribosome-binding sites are disclosed, for example, by Gold et al. (Ann. Rev. Microbiol. 35:365-404, 1981). The selection of control sequences, expression vectors, transformation methods, and the like, are dependent on the type of host cell used to express the gene. As used herein, "cell", "cell line", and "cell culture" may be used interchangeably and all such designations include progeny. Thus, the words "transformants" or "transformed cells" include the primary subject cell and cultures derived therefrom, without regard to the number of transfers. It is also understood that all progeny may not be precisely identical in DNA content, due to deliberate or inadvertent mutations. However, as defined, mutant progeny have the same functionality as that of the originally transformed cell.

Host cells which may be used in the expression systems of the present invention are not strictly limited, provided that they are suitable for use in the expression of the polypeptide of interest. Suitable hosts may also include eukaryotic cells. Preferred eukaryotic hosts include, for example, yeast, fungi, plant cells, algae cells, insect cells, mammalian cells either *in vivo*, or in tissue culture.

Another host is an insect cell, for example the *Drosophila* larvae. Using insect cells as hosts, the *Drosophila* alcohol dehydrogenase promoter can be used (Rubin, Science 240:1453-1459, 1988). Alternatively, baculovirus vectors can be engineered to express large amounts of polypeptide of the invention in insect cells (Jasny, Science 238:1653, 1987; Miller et al., In: Genetic Engineering, Vol. 8, Plenum, Setlow et al., eds., pp. 277-297, 1986).

Any of a series of yeast expression systems can be utilized which incorporate promoter and termination elements from the actively expressed sequences coding for glycolytic enzymes that are produced in large quantities when yeast are grown in mediums rich in glucose. Known glycolytic gene sequences can also provide very efficient transcriptional control signals. A number of recombinant DNA strategies exist utilizing strong promoter sequences and high copy number plasmids, which can be utilized for production of the desired proteins in yeast. Several possible vector systems are available for the expression in a mammalian host.

In one embodiment of the invention, the eukaryotic host cells are yeast cells, such as *Saccharomyces cerevisiae*. In another embodiment of the invention, the host cells are algae cells, for example *Chlamydomonas reinhardtii* cells.

Preferred eukaryotic plasmids include, for example, BPV, vaccinia, SV40, 2-micron circle, and the like, or their derivatives. Such plasmids are well known in the art (Botstein et al., Miami Wntr. Symp. 19:265-274, 1982; Broach, In: "The Molecular Biology of the Yeast Saccharomyces: Life Cycle and Inheritance", Cold Spring Harbor Laboratory, Cold Spring Harbor, NY, p. 445-470, 1981; Broach, Cell 28:203-204, 1982; Bollon et al., J. Clin. Hematol. Oncol. 10:39-48, 1980; Maniatis, In: Cell Biology: A Comprehensive Treatise, Vol. 3, Gene Sequence Expression, Academic Press, NY, pp. 563-608, 1980).

A wide variety of transcriptional and translational regulatory sequences may be employed, depending upon the nature of the host. The transcriptional and translational regulatory signals may be derived from viral sources, such as adenovirus, bovine papilloma virus, cytomegalovirus, simian virus, or the like, where the regulatory signals are associated with a particular gene sequence which has a high level of expression. Alternatively, promoters from mammalian expression products, such as actin, collagen, myosin, and the like, may be employed. Transcriptional initiation regulatory signals may be selected which allow for repression or activation, so that expression of the gene sequences can be modulated. Of interest are regulatory signals which are temperature-sensitive so that by varying the temperature, expression can be repressed or initiated, or are subject to chemical (such as metabolite) regulation.

Expression of polypeptides of the invention in eukaryotic hosts requires the use of eukaryotic regulatory regions. Such regions will, in general, include a promoter region sufficient to direct the initiation of RNA synthesis. Eukaryotic promoters include, for example, the promoter of the mouse metallothionein I gene sequence (Hamer et al., J. Mol. Appl. Gen. 1:273-288, 1982); the TK promoter of Herpes virus (McKnight, Cell 31:355-365, 1982); the SV40 early promoter (Benoist et al., Nature (London) 290:304-31, 1981); and the yeast gal4 gene sequence promoter (Johnston et al., Proc. Natl. Acad. Sci. (USA) 79:6971-6975, 1982; Silver et al., Proc. Natl. Acad. Sci. (USA) 81:5951-5955, 1984).

Translation of eukaryotic mRNA is initiated at the codon which encodes the first methionine. For this reason, it is preferable to ensure that the linkage between a eukaryotic promoter and a DNA sequence which encodes a polypeptide of the invention (or a functional derivative thereof) does not contain any intervening codons which are capable of encoding a methionine (*i*.*e*., AUG). The presence of such codons results either in the formation of a fusion protein (if the AUG codon is in the same reading frame as the polypeptide of the invention coding sequence) or a frame-shift mutation (if the AUG codon is not in the same reading frame as the polypeptide of the invention coding sequence).

A nucleic acid molecule encoding a polypeptide of the invention and an operably linked promoter may be introduced into a recipient prokaryotic or eukaryotic cell either as a non-replicating DNA or RNA molecule, which may either be a linear molecule or, more preferably, a closed covalent circular molecule. Since such molecules are incapable of autonomous replication, the expression of the gene may occur through the transient expression of the introduced sequence. Alternatively, permanent expression may occur through the integration of the introduced DNA sequence into the host chromosome.

A vector may be employed which is capable of integrating the desired gene sequences into the host cell chromosome. Cells which have stably integrated the introduced DNA into their chromosomes can be selected by also introducing one or more markers which allow for selection of host cells which contain the expression vector. The marker may provide for prototrophy to an auxotrophic host, biocide resistance, *e.g*., antibiotics, or heavy metals, such as copper, or the like. The selectable marker gene sequence can either be directly linked to the DNA gene sequences to be expressed, or introduced into the same cell by co-transfection. Additional elements may also be needed for optimal synthesis of mRNA. These elements may include splice signals, as well as transcription promoters, enhancers, and termination signals. cDNA expression vectors incorporating such elements include those described by Okayama (Mol. Cell. Biol. 3:280, 1983).

The introduced nucleic acid molecule can be incorporated into a plasmid or viral vector capable of autonomous replication in the recipient host. Any of a wide variety of vectors may be employed for this purpose. Factors of importance in selecting a particular plasmid or viral vector include: the ease with which recipient cells that contain the vector may be recognized and selected from those recipient cells which do not contain the vector; the number of copies of the vector which are desired in a particular host; and whether it is desirable to be able to "shuttle" the vector between host cells of different species.

Once the vector or nucleic acid molecule containing the construct(s) has been prepared for expression, the DNA construct(s) may be introduced into an appropriate host cell by any of a variety of suitable means, *i*.*e*., transformation, transfection, conjugation, protoplast fusion, electroporation, particle gun technology, calcium phosphate-precipitation, direct microinjection, and the like. After the introduction of the vector, recipient cells are grown in a selective medium, which selects for the growth of vector-containing cells. Expression of the cloned gene(s) results in the production of a polypeptide of the invention, or fragments thereof. This can take place in the transformed cells as such, or following the induction of these cells to differentiate. A variety of incubation conditions can be used to form the peptide of the present invention. The most preferred conditions are those which mimic physiological conditions.

### Preparation methods

A variety of methodologies known in the art can be utilized to obtain the polypeptides of the present invention. The polypeptides may be purified from tissues or cells that naturally produce the polypeptides. Alternatively, the above-described isolated nucleic acid fragments could be used to express the recombinant polypeptides of the invention in any organism.

In one embodiment, the invention is therefore directed to a method for preparing a polypeptide, comprising recombinantly expressing a nucleic acid molecule or vector according to the invention in a host cell and obtaining therefrom the polypeptide. This method may include the cultivation of a host cell containing the nucleic acid or vector of the invention. The cultivation of the host cell may be done in the presence of a chromophore to yield the fluorescent adduct of the invention. Accordingly, in one embodiment, the host cell is capable of synthesizing the chromophore or the chromophore has been added to the growth medium. The synthesis of the chromophore may be achieved by co-transfecting the cells with nucleic acid molecules that encode enzymes necessary for chromophore synthesis. In one embodiment of the invention, the host cell is capable of synthesizing tetrapyrrole chromophores, such as heme.

As a further alternative the polypeptides of the invention may be synthesized using an automated polypeptide synthesizer.

One skilled in the art can readily follow known methods for isolating proteins in order to obtain the polypeptides free of natural contaminants. These include, but are not limited to: size-exclusion chromatography, HPLC, ion-exchange chromatography, and immunoaffinity chromatography.

The invention further features a method for the preparation of a fluorescent adduct, comprising contacting a polypeptide according to the invention with a chromophore.

In the methods of the invention, the chromophore may be a bilin, for example selected from the group consisting of phycoerythrobilin, phycocyanobilin, phytochromobilin, phycourobilin, phycoviolobilin, dihydrobiliverdin, biliverdin, bilirubin and derivatives thereof.

### Utility

The present invention also relates to the use of the invented polypeptides and fluorescent adducts as fluorescence markers. This use encompasses *in vivo* as well as *in vitro* marker.

Possible *in vivo* uses include the use as a reporter for gene expression in cells. In such applications the present invention may be used to study promoter activity. In other applications, the polypeptides and fluorescent proteins/adducts of the present invention may be used as FRET (fluorescence resonance energy transfer) reporters, for example by combination with orange fluorescent protein (OFP; λem= 573 nm) to investigate protein-protein interactions. In other applications, the polypeptides and fluorescent proteins/adducts of the present invention may function as molecular switches, i.e. molecules that cycle between two stable states depending on the presence or absence of the chromophore ligand. In specific embodiments, the two states are clearly distinguishable, for example in that one state is fluorescent, whereas the other shows no fluorescence.

For such *in vivo* applications, *Escherichia coli*, *Bacillus subtilis*, *Saccharomyces cerevisiae* and mammalian cells that express the polypeptides of the invention and, optionally, are also capable of synthesizing the chromophore, for example PEB, may be used.

The invention also includes kits that comprise an expression plasmid for the polypeptides of the invention optionally including a multiple cloning site in order to allow the practitioner the generation of N-terminal or C-terminal fusion proteins. The kits may further contain cells or strains that are capable of synthesizing a chromophore that is bound by the polypeptide to yield a fluorescent adduct.

The *in vitro* use of the polypeptide or the fluorescent adduct according to the invention, includes the use as a detectable label.

In such applications, the polypeptide or the fluorescent adduct according to the invention may be coupled to a primary or secondary antibody or other binding molecules, such as avidin, streptavidin and neutravidin. Suitable methods for the coupling are known in the art.

Possible fields of application for the polypeptides or the fluorescent adducts according to the invention and molecules comprising them, are thus flow cytometry (FACS analysis), fluorescence microscopy, Western Blots and immunoassays.

The polypeptide or the fluorescent adduct according to the invention can also be used to visualize protein-protein interactions, protein-ligand interactions and protein trafficking.

Detection molecules that comprise the polypeptide or the fluorescent adduct according to the invention may further be used for analyte detection, for example in a sample.

The present invention thus also includes detection molecules, detection reagents or conjugates that comprise the polypeptide(s) or fluorescent proteins/adducts of the invention as well as kits comprising these molecules, reagents or conjugates.

In a further aspect, the invention thus also covers a method for detecting the presence of an analyte in a sample, the method comprising:
(a) contacting a sample with a detection reagent that specifically binds the analyte, wherein the detection reagent comprises a polypeptide or fluorescent adduct according to the invention;
(b) exposing the sample to light that causes the fluorescent adduct to emit light;
(c) detecting the emitted light, thereby detecting the presence of the analyte.

The method may include additional washing steps to remove unbound detection reagent prior to the detection step. The method may also be carried out in a sandwich format, where the analyte or detection reagent:analyte complex is immobilized on a solid support by using a capture molecule that specifically binds the analyte without impairing the ability of the detection reagent to bind the analyte.

In certain embodiments of the invention, the method includes an immunoassay. The detection reagent may comprise an antibody, antibody fragment or antibody-like molecule.

In the methods of the invention, the light used for excitation of the fluorescent protein/adduct may have an excitation wavelength of about 610-615 nm and/or the emitted light may have a wavelength of about 620-625 nm.

In certain embodiments of the invention, the invented methods thus include exposing the sample to light of a wavelength that excites the fluorescent protein/adduct of the invention, for example light of a wavelength of 610-615 nm, and/or detecting light emitted by the fluorescent protein/adduct after excitation, wherein the detected/emitted light may, for example, have a wavelength of about 620-625 nm.

The present invention also encompasses kits that provide the polypeptides, fluorescent adducts, hybrid molecules, fusion proteins, detection reagents, nucleic acids, vectors and/or host cells of the invention in a ready-to-use format. Such kits may for example include the polypeptides or fluorescent adducts of the invention linked to a binding molecule that allows the versatile coupling to detection reagents of choice. In such kits the binding molecule may be avidin, streptavidin or neutravidin as well as biotin-binding fragments thereof that allow conjugation of the polypeptides or fluorescent adducts of the invention to biotinylated detection reagents, for example antibodies or nucleic acid probes. In another embodiment, such kits include a conjugate of a polypeptide or fluorescent adduct of the invention and a (secondary) antibody. The secondary antibody may have specificity for the IgG molecules of a certain species, such as human, mouse, sheep, goat, rabbit etc. and thus allow detection in a conventional immunoassay utilizing an analyte specific primary antibody and a detectable secondary antibody specific for the primary antibody. In still further embodiments of the invention, the kits of the invention may comprise a nucleic acid of the invention, for example ligated into an expression vector, and, optionally, a host cell strain that is suitable for the expression of the polypeptides encoded by the nucleic acids. Such kits allow the practitioner to clone a gene of choice into the MCS of the vector and then express the polypeptide encoded by the gene of choice as a fusion protein with the fluorescent polypeptide of the invention, optionally, in the host cells comprised within the kit.

The inventions illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the appended claims and non-limiting examples. In addition, where features or aspects of the invention are described in terms of Markush groups, those skilled in the art will recognize that the invention is also thereby described in terms of any individual member or subgroup of members of the Markush group.

### Examples

The present inventions will be explained in more detail in the following examples. However, the examples are only used for illustration and do not limit the scope of the present invention.

### Reagents

Unless otherwise specified, all chemical reagents were ACS grade or better. Enzymes were obtained from Fermentas or Sigma. Strep-Tactin Sepharose and the expression vector pASKIBA45+ were obtained from IBA GmbH (Goettingen, Germany).

### Instruments

Absorption was determined with an Agilent Technologies 8453 spectrophotometer. Fluorescence measurements were performed with an AmincoBowman AB2 spectrofluorimeter.

### Example 1: PEB isolation from Porphyridium cruentum

*P. cruentum* cells were grown in liquid culture under light and shaking at 18°C. The cell pellet was extracted with acetone until the supernatant no longer looked colored to the eye. Subsequently, the pellet was resuspended in methanol containing 2 mg/ml HgCl₂. The mixture was stirred at 45 °C for 16 hours and kept under in dark to minimize photodamage to pigments. Cell debris was separated from the bilin containing supernatant with a sintered glass filter. HgCl₂ was removed by adding 2-mercaptoethanol (2 µl/ml) to the filtrate. After centrifugation the supernatant was diluted 10 times with 0.1% aqueous trifluoroacetic acid (TFA) and loaded onto a Sep-pak column equilibrated with 10% Methanol/90% of 0.1 % TFA. After washing with 0.1% TFA, pigments were eluted using 60% CH₃CN/40% of 0.1% TFA and dried in a speed vac. The 3E-isomer of PEB was further purified from the crude bilin mixture by C18 reverse phase HPLC using a Agilent 1100 series liquid chromatograph and a Phenomenex Ultracarb 5µ ODS (20) 10 mm x 250 mm with a 10 mm x 30 mm guard column. The solvent system used was 50:50 Acetone:20 mM formic acid with a flow rate of 2 ml/min. Eluted PEB was monitored at 380 nm and 560 nm, concentrated via Sep-pak as described above and dried in a speed vac. Before use, PEB was dissolved in dimethyl sulfoxide to a concentration of about 2 mM. The extinction coefficient of 25,200 M⁻¹ cm⁻¹ at 591 nm for 3E-PEB in 5% HCl/95% MeOH was used to estimate the concentration spectrophotometrically.

### Example 2: Expression of cpeT

The *cpeT* gene coding for a T-type phycobiliprotein lyase was identified in the genome of the cyanophage Syn9 (Weigele, P.R., et al., Genomic and structural analysis of Syn9, a cyanophage infecting marine Prochlorococcus and Synechococcus. Environ Microbiol, 2007. 9(7): p. 1675-95). The *cpeT* coding region (SEQ ID NO:1) was PCR-amplified from Syn9 phage lysate with primers encompassing recognition sites for EcoRI (5'-ggaattcgcatatgaacctggagccccagag-3'; SEQ ID NO:13) and XhoI (5'-ccgctcgagctattcgccctctcggac-3'; SEQ ID NO:14). Subsequently, the EcoRI-XhoI-*cpeT* fragment was cloned into expression vector pASKIBA45+ (IBA GmbH, Goettingen, Germany) (Fig. 3).

Subsequently, CpeT was recombinant produced in *E. coli* BL21(DE3) with an N-terminal *Strep*-tag® and purified via affinity chromatography performed according to the protocol supplied by the manufacturer. Migration of the purified protein during SDS-PAGE was in agreement with its calculated molecular masses of about 21 kDa (_{Strep}CpeT) (Fig. 4).

### Example 3: Fluorescent adduct formation

CpeT and PEB were incubated in a 1:1 ratio in buffer A (50 mM Na-phosphate, 100 mM NaCl pH 7) at room temperature. Incubation of CpeT with the pinkish, not fluorescent linear tetrapyrrole PEB resulted nearly immediately in a complex with an absorption maximum at 612 nm and a blue color (Fig. 5). The CpeT:PEB complex exhibited a fluorescence emission maximum at 621 nm when excitated in a range from 550 nm to 612 nm (Fig. 6). After incubation of CpeT with PEB a second affinity chromatography was performed to remove the free chromophor.

Spectroscopic data obtained after purification were indistinguishable from those before affinity chromatography indicating the formation of an extremely stable protein-chromophore-complex. This was verified by the detection of CpeT:PEB after SDS-PAGE by Zinc-induced red fluorescence (Fig. 6).

The fact that PEB is not covalently bound to CpeT was demonstrated by treatment with 1M NaCl or acidic urea, which resulted in the release of PEB from the protein (data not shown).

The advantageous excitation and emission maxima and the small size of CpeT render the fluorescent complex highly useful as a fluorescence marker. The non-covalent binding of the chromophore allows to use CpeT:PEB as a molecular switch. Possible application strategies have been described above.

### Example 4: Tryptic digestion

Optimizing Syn9 CpeT considering its molecular weight was investigated via tryptic digest of the CpeT:PEB complex (Fig. 7): after addition of 150000 units trypsin to 1 mg CpeT in buffer A tryptic digestion was performed at room temperature for 60 min. During incubation samples were taken and analyzed in a Coomassie stained SDS-Gel and in a zinc-induced red fluorescence blot.

A chromopeptide of about 12 kDA was generated as detected by zinc-induced red fluorescence of the chromophore, indicating that truncated versions of CpeT are still capable of binding PEB, resulting in a fluorescent complex.

## Claims

1. An isolated polypeptide comprising, consisting essentially of or consisting of the amino acid sequence set forth in any one of SEQ ID Nos. 7-12 and mutants, variants and fragments thereof.

2. The isolated polypeptide of claim 1, wherein the polypeptide
(a) comprises, consists essentially of or consists of at least 30, 35, 40, 45, 50, 60, 70, 80, 90, 100, 120, or 150 contiguous amino acids of SEQ ID Nos. 7-12;
(b) comprises an amino acid sequence that is about 60, about 70, about 80, about 90, about 95, about 97, about 98 or about 99 % identical to the amino acid sequences set forth in any one of SEQ ID Nos. 7-12;
(c) wherein the polypeptide is derived from a cyanophage; or
(d) wherein the polypeptide has phycobiliprotein lyase activity

3. A fluorescent adduct comprising the isolated polypeptide of claims 1 or 2 and a chromophore moiety.

4. The fluorescent adduct of claim 3, wherein the chromophore moiety is
(a) a tetrapyrrole;
(b) an open chain tetrapyrrole;.
(c) a bilin;
(d) a phycobilin;
(e) selected from the group consisting of phycoerythrobilin, phycocyanobilin, phytochromobilin, phycourobilin, phycoviolobilin, dihydrobiliverdin, biliverdin, bilirubin and derivatives thereof; or
(f) covalently or non-covalently bound to the polypeptide;

5. The fluorescent adduct according to claim 3 or 4, wherein the fluorescent adduct has an excitation spectrum maximum and/or an emission spectrum maximum in the region of about 600 to 700 nm.

6. A hybrid molecule comprising the polypeptide of claim 1 or 2 or the fluorescent adduct of any one of claims 3 - 5 linked to a biomolecule.

7. The hybrid molecule of claim 6, wherein
(a) the linkage is a covalent bond;
(b) the biomolecule is selected from the group consisting of a polypeptide, a protein, a carbohydrate, a lipid, and a nucleic acid;
(c) the hybrid molecule is a hybrid protein; or
(d) the biomolecule is a polypeptide or protein selected from the group consisting of a signalling protein, a cell-surface receptor, an antibody, an antibody fragment or an antibody-like molecule.

8. An isolated or synthesized nucleic acid molecule selected from the group consisting of:
(a) nucleic acid molecules comprising, consisting essentially of or consisting of the nucleotide sequence set forth in any one of SEQ ID Nos. 1-6 or complements thereof;
(b) nucleic acid molecules encoding a polypeptide sequence having the amino acid sequence set forth in any one of SEQ ID Nos. 7-12 or complements thereof;
(c) nucleic acid molecules encoding the polypeptide of any one of claims 1-9;
(d) nucleic acid molecules comprising, consisting essentially of or consisting of a nucleotide sequence that has at least 50, 60, 70, 80, 90, 95, 98 or 99 % sequence identity to the nucleic acid molecules of (a) - (c); and
(e) nucleic acid molecules comprising:
(i) a first coding sequence selected from the group consisting of the nucleic acid molecules of the nucleic acid molecules of (a)-(d); and
(ii) a second coding sequence that encodes a selected polypeptide, wherein the first coding sequence is fused to the second coding sequence such that expression of the fused sequence yields a hybrid protein in which the polypeptide encoded by the first coding sequence is fused to the polypeptide encoded by the second coding sequence

9. A vector comprising the nucleic acid molecule of claim 8.

10. A host cell comprising the nucleic acid according to claim 8 or the vector according to claim 9.

11. Method for preparing a recombinant polypeptide, comprising expressing a nucleic acid according to claim 8 or a vector according to claim 9 in a host cell and obtaining therefrom the polypeptide, wherein method optionally further comprises cultivating the host cell in the presence of a chromophore to yield a fluorescent polypeptide.

12. Method for the generation of a fluorescent adduct, comprising contacting a polypeptide according to claim 1 or 2 with a chromophore.

13. Use of the polypeptide according to claim 1 or 2 or the fluorescent adduct according to any one of claims 3 - 5 as a detectable label, a fluorescence marker, a reporter for gene expression in cells, a molecular switch or a FRET reporter.

14. A method of detecting the presence of an analyte in a sample, comprising:
(a) contacting a sample with a detection reagent that specifically binds the analyte, wherein the detection reagent comprises a fluorescent adduct according to any one of claims 3-5;
(b) exposing the sample to light that causes the fluorescent adduct to emit light;
(c) detecting the emitted light, thereby detecting the presence of the analyte.

15. Kit comprising
(a) the hybrid molecule according to 6 or 7; or
(b) the vector according to claim 9 and, optionally, a host cell strain.


Sequence listing

<110>  Ruhr-Universität-Bochum

<120>  Fluorescent proteins

<130>  P40723

<160>  14

<170>  PatentIn version 3.3

<210>  1
<211>  486
<212>  DNA
<213>  Cyanophage Syn9

<400>  1
atgaacctgg agccccagag tgtaaaatcc acgataacta aatttgaaag ttatctttta      60

ggaaaatata acaactggaa gcaagcacag tcctacccta caatcttttc ccaagtttgt     120

attctttggg aaaagattga cggtgggtat cattcttgtc aatggtatag acgagatcct     180

agaaatccat atcgagaaaa atatcacaag atagaagtaa ttgacgaaaa cacagtcatc     240

gttaaaaatt atcatctaga ctggacaaga cacgaagatt gtgatatgat atttaccttc     300

ttcaacaatc aatgggatgg taaattatta ggagacaaat gtattgttag aggcaatgca     360

agagtagtgt ctgaagtgca tctaactaag gtgggtctcg aatcaagaga caaaggtgtt     420

gatgaagacg gaaacaaggt tttcggaggt tgggacctat ataaatttgt ccgagagggc     480

gaatag                                                                486

<210>  2
<211>  540
<212>  DNA
<213>  Cyanophage Syn1

<400>  2
atgattgatc aattttatga atggtttgaa ggaagatata acaataaaat tcaagcattt      60

tcgcatccat ctaaatttgc atacattatt gttgagcatc gtgctataaa caatcatgga     120

ctgttttatg gcgagcaagc atattttaat caaactcgaa caccatatag acaatttcta     180

ttacaaattt ctgaatgtca tggtaaagtt gttgtaagat caatggaacc aaaaaataag     240

tctacttata ctggtttcaa aaatcttaat cttatatctg gtagtccctt gacatacaag     300

aaaggatgtg atacaatatt tacttggcgt cctgatgcta accagtttat tggtgaaatt     360

gaaccaggat gtaactgtaa agtaaaatgg ggagacaaag attcttatct ccaaaatatt     420

gcagcattgg gagatgggtg gtataatgta gaagacaagg ggtttgatcc tgaaactaat     480

cagcaactct ggggttccag acatggcaga tttatgttca agaaagaaac atcacaatag     540

<210>  3
<211>  450
<212>  DNA
<213>  Cyanophage Syn19

<400>  3
atgagctttg agtcttactt gcttggacat tggactaacg tatcacaagc acaatccgat      60

cctctattat tctcttccgt agaacttgta tgggatcgga ttcctggtgg attgcgttct     120

aaaaactttt atagaaagac ccctaactct ccatataggg aaagatatca caagattaat     180

caagtatcag ataccaaagt aattgttgag aactaccaca ctgactggac tagatcagaa     240

aattgtgata tgatgtttgt atacaacggc gagcaatgga taggttctgt tgttggtgat     300

agttgtatta gttctgatgg aaaccgtgtt cactctgaaa tgaaattgtt tggcagcaaa     360

attcatactt gtgatcaagg atatgattct gatggtaata tgatttgggg ttctaaaaac     420

ccgtataaat ttaagagaat gggcgaatag                                      450

<210>  4
<211>  528
<212>  DNA
<213>  Cyanophage S-PM2

<400>  4
atgattgaaa cattttacga atggtttgaa ggaaaattca acaatagaat tcaagcattt      60

acatatccat ctagatatgc atacatagtg gttgaacata gaaaagtaaa taaccatggt     120

ttgttttatg gggaacaagc atacttcaat aaaacactaa aaccatatag acaatttatc     180

ttacaaatta ccgagagtca cgataatatc attgtaagaa attatgaggt taaagataaa     240

tcattgcatc ttggattcaa aaatttaaat ttgatttctg gtaatgcatt gacttacaaa     300

cctggatgtg atacaatatt tacataccat ggtgatcact tcgaaggtca tatcgaagag     360

ggatgtaatt gcctagtcaa gtggggtgaa aaagattcat atcttaaaaa ctctgcatat     420

ataggtgatg ggtggtataa tgtagaggat aaaggatttg atccagaaac ccatactcaa     480

ctctggggat ctaaatttgg tcattttgaa tttaaaaaaa tcgcctga                  528

<210>  5
<211>  459
<212>  DNA
<213>  Cyanophage S-SSM5

<400>  5
atgtctatac agattgatga ttatctttta ggacattgga caaatagaca tcaagcacaa      60

tccgctccac atcattattc cagtatagaa attgtatgga gtaaagttga gggtggttat     120

cattcaaaga attattatcg atcacaagga cctgataatc cctacagaga aaggtaccat 180

aaaactgatg tgatatcaga tgataaaata atttttaaaa attacaattt agattggaca 240

agatcggaaa attgtgatat gatgtttaca ttcgatggca ctgcatggca cggacactta 300

gtcggtgata aatgcactgg tgccatggga tatcgtgttg tttctgagat tagtctttat 360

ggcgagaaac tacatagtaa agatcaaggt tatgatgaaa aaggtgatat gatatgggga 420

agtaacgcat tatataaatt cattcgctgg ggagaatag 459

<210> 6
<211> 457
<212> DNA
<213> Cyanophage P-RSM4

<400> 6
atgattgacg attatattat aggacactgg acaaatagat atcaagcaca atcagcacct 60

caccatttct ctactgttga aactatatgg gaaaaggtag agggtggtta tcattcaaag 120

aatttttata gaagagacgg tgctaataaa ccatatagag aaagatatca caaggttaat 180

gtaatatcct ttgataaatt aatttttgaa aattataact tagactggac aagatcagag 240

aattgtgata tgatattcac attcgatggt gaagcatggc atggacacct agtaggggat 300

aaatgcactg gtgccaaagg atataagatt gtatctgaga tcactcttta cgggaatcga 360

ctgcatagta tggatcaagg atatgatgat aaaggtaaca tggtatgggg tagtgagggc 420

atttataaat tcactcgctg tatcgaccct aaataaa 457

<210> 7
<211> 161
<212> PRT
<213> Cyanophage Syn9

<400> 7

Met Asn Leu Glu Pro Gln Ser Val Lys Ser Thr Ile Thr Lys Phe Glu
1               5                   10                  15

Ser Tyr Leu Leu Gly Lys Tyr Asn Asn Trp Lys Gln Ala Gln Ser Tyr
            20                  25                  30

Pro Thr Ile Phe Ser Gln Val Cys Ile Leu Trp Glu Lys Ile Asp Gly
        35                  40                  45

Gly Tyr His Ser Cys Gln Trp Tyr Arg Arg Asp Pro Arg Asn Pro Tyr
    50                  55                  60

Arg Glu Lys Tyr His Lys Ile Glu Val Ile Asp Glu Asn Thr Val Ile

65                  70                  75                  80

Val Lys Asn Tyr His Leu Asp Trp Thr Arg His Glu Asp Cys Asp Met
                85                  90                  95

Ile Phe Thr Phe Phe Asn Asn Gln Trp Asp Gly Lys Leu Leu Gly Asp
            100                 105                 110

Lys Cys Ile Val Arg Gly Asn Ala Arg Val Val Ser Glu Val His Leu
        115                 120                 125

Thr Lys Val Gly Leu Glu Ser Arg Asp Lys Gly Val Asp Glu Asp Gly
    130                 135                 140

Asn Lys Val Phe Gly Gly Trp Asp Leu Tyr Lys Phe Val Arg Glu Gly
145                 150                 155                 160

Glu

<210>  8
<211>  179
<212>  PRT
<213>  Cyanophage Syn1

<400>  8

Met Ile Asp Gln Phe Tyr Glu Trp Phe Glu Gly Arg Tyr Asn Asn Lys
1               5                   10                  15

Ile Gln Ala Phe Ser His Pro Ser Lys Phe Ala Tyr Ile Ile Val Glu
            20                  25                  30

His Arg Ala Ile Asn Asn His Gly Leu Phe Tyr Gly Glu Gln Ala Tyr
        35                  40                  45

Phe Asn Gln Thr Arg Thr Pro Tyr Arg Gln Phe Leu Leu Gln Ile Ser
    50                  55                  60

Glu Cys His Gly Lys Val Val Val Arg Ser Met Glu Pro Lys Asn Lys
65                  70                  75                  80

Ser Thr Tyr Thr Gly Phe Lys Asn Leu Asn Leu Ile Ser Gly Ser Pro
                85                  90                  95

Leu Thr Tyr Lys Lys Gly Cys Asp Thr Ile Phe Thr Trp Arg Pro Asp
            100                 105                 110

Ala Asn Gln Phe Ile Gly Glu Ile Glu Pro Gly Cys Asn Cys Lys Val
        115                 120                 125

Lys Trp Gly Asp Lys Asp Ser Tyr Leu Gln Asn Ile Ala Ala Leu Gly
    130                 135                 140

Asp Gly Trp Tyr Asn Val Glu Asp Lys Gly Phe Asp Pro Glu Thr Asn
145                 150                 155                 160

Gln Gln Leu Trp Gly Ser Arg His Gly Arg Phe Met Phe Lys Lys Glu
                165                 170                 175

Thr Ser Gln

<210>  9
<211>  149
<212>  PRT
<213>  Cyanophage Syn19

<400>  9

Met Ser Phe Glu Ser Tyr Leu Leu Gly His Trp Thr Asn Val Ser Gln
1               5                   10                  15

Ala Gln Ser Asp Pro Leu Leu Phe Ser Ser Val Glu Leu Val Trp Asp
            20                  25                  30

Arg Ile Pro Gly Gly Leu Arg Ser Lys Asn Phe Tyr Arg Lys Thr Pro
        35                  40                  45

Asn Ser Pro Tyr Arg Glu Arg Tyr His Lys Ile Asn Gln Val Ser Asp
    50                  55                  60

Thr Lys Val Ile Val Glu Asn Tyr His Thr Asp Trp Thr Arg Ser Glu
65                  70                  75                  80

Asn Cys Asp Met Met Phe Val Tyr Asn Gly Glu Gln Trp Ile Gly Ser
                85                  90                  95

Val Val Gly Asp Ser Cys Ile Ser Ser Asp Gly Asn Arg Val His Ser
            100                 105                 110

Glu Met Lys Leu Phe Gly Ser Lys Ile His Thr Cys Asp Gln Gly Tyr
        115                 120                 125

Asp Ser Asp Gly Asn Met Ile Trp Gly Ser Lys Asn Pro Tyr Lys Phe
    130                 135                 140

Lys Arg Met Gly Glu
145

<210>  10
<211>  175
<212>  PRT
<213>  Cyanophage S-PM2

<400>  10

Met Ile Glu Thr Phe Tyr Glu Trp Phe Glu Gly Lys Phe Asn Asn Arg
1               5                   10                  15

Ile Gln Ala Phe Thr Tyr Pro Ser Arg Tyr Ala Tyr Ile Val Val Glu
            20                  25                  30

His Arg Lys Val Asn Asn His Gly Leu Phe Tyr Gly Glu Gln Ala Tyr
        35                  40                  45

Phe Asn Lys Thr Leu Lys Pro Tyr Arg Gln Phe Ile Leu Gln Ile Thr
    50                  55                  60

Glu Ser His Asp Asn Ile Ile Val Arg Asn Tyr Glu Val Lys Asp Lys
65                  70                  75                  80

Ser Leu His Leu Gly Phe Lys Asn Leu Asn Leu Ile Ser Gly Asn Ala
                85                  90                  95

Leu Thr Tyr Lys Pro Gly Cys Asp Thr Ile Phe Thr Tyr His Gly Asp
            100                 105                 110

His Phe Glu Gly His Ile Glu Glu Gly Cys Asn Cys Leu Val Lys Trp
        115                 120                 125

Gly Glu Lys Asp Ser Tyr Leu Lys Asn Ser Ala Tyr Ile Gly Asp Gly
    130                 135                 140

Trp Tyr Asn Val Glu Asp Lys Gly Phe Asp Pro Glu Thr His Thr Gln
145                 150                 155                 160

Leu Trp Gly Ser Lys Phe Gly His Phe Glu Phe Lys Lys Ile Ala
                165                 170                 175

<210>  11
<211>  152
<212>  PRT
<213>  Cyanophage S-SSM5

<400>  11

Met Ser Ile Gln Ile Asp Asp Tyr Leu Leu Gly His Trp Thr Asn Arg
1               5                   10                  15

His Gln Ala Gln Ser Ala Pro His His Tyr Ser Ser Ile Glu Ile Val
            20                  25                  30

Trp Ser Lys Val Glu Gly Gly Tyr His Ser Lys Asn Tyr Tyr Arg Ser
        35                  40                  45

Gln Gly Pro Asp Asn Pro Tyr Arg Glu Arg Tyr His Lys Thr Asp Val
    50                  55                  60

Ile Ser Asp Asp Lys Ile Ile Phe Lys Asn Tyr Asn Leu Asp Trp Thr
65                  70                  75                  80

Arg Ser Glu Asn Cys Asp Met Met Phe Thr Phe Asp Gly Thr Ala Trp
                85                  90                  95

His Gly His Leu Val Gly Asp Lys Cys Thr Gly Ala Met Gly Tyr Arg
            100                 105                 110

Val Val Ser Glu Ile Ser Leu Tyr Gly Glu Lys Leu His Ser Lys Asp
        115                 120                 125

Gln Gly Tyr Asp Glu Lys Gly Asp Met Ile Trp Gly Ser Asn Ala Leu
    130                 135                 140

Tyr Lys Phe Ile Arg Trp Gly Glu
145                 150

<210>  12
<211>  151
<212>  PRT
<213>  Cyanophage P-RSM4

<400>  12

Met Ile Asp Asp Tyr Ile Ile Gly His Trp Thr Asn Arg Tyr Gln Ala
1               5                   10                  15

Gln Ser Ala Pro His His Phe Ser Thr Val Glu Thr Ile Trp Glu Lys
            20                  25                  30

Val Glu Gly Gly Tyr His Ser Lys Asn Phe Tyr Arg Arg Asp Gly Ala
        35                  40                  45

Asn Lys Pro Tyr Arg Glu Arg Tyr His Lys Val Asn Val Ile Ser Phe
    50                  55                  60

Asp Lys Leu Ile Phe Glu Asn Tyr Asn Leu Asp Trp Thr Arg Ser Glu
65                  70                  75                  80

Asn Cys Asp Met Ile Phe Thr Phe Asp Gly Glu Ala Trp His Gly His
                85                  90                  95

Leu Val Gly Asp Lys Cys Thr Gly Ala Lys Gly Tyr Lys Ile Val Ser
            100                 105                 110

Glu Ile Thr Leu Tyr Gly Asn Arg Leu His Ser Met Asp Gln Gly Tyr
        115                 120                 125

Asp Asp Lys Gly Asn Met Val Trp Gly Ser Glu Gly Ile Tyr Lys Phe
    130                 135                 140

Thr Arg Cys Ile Asp Pro Lys
145                 150

<210>  13
<211>  31
<212>  DNA
<213>  Artificial

<220>
<223>  PCR primer

<400>  13
ggaattcgca tatgaacctg gagccccaga g                                    31

<210>  14
<211>  27
<212>  DNA
<213>  Artificial

<220>
<223>  PCR primer

<400>  14
ccgctcgagc tattcgccct ctcggac                                         27